(19) Europäisches Patentamt
European Patent Office
Office européen des brevets

(11) **EP 4 098 127 A1**

(12) **EUROPEAN PATENT APPLICATION**
published in accordance with Art. 153(4) EPC

(43) Date of publication:
**07.12.2022 Bulletin 2022/49**

(21) Application number: **21747234.9**

(22) Date of filing: **29.01.2021**

(51) International Patent Classification (IPC):
*A23J 3/00* (2006.01)  *A23J 3/14* (2006.01)
*A23J 3/22* (2006.01)  *C07K 14/00* (2006.01)
*C07K 14/415* (2006.01)  *C07K 14/435* (2006.01)
*A23L 13/00* (2016.01)

(52) Cooperative Patent Classification (CPC):
**A23J 3/00; A23J 3/14; A23J 3/22; A23L 13/00;
C07K 14/00; C07K 14/415; C07K 14/435**

(86) International application number:
**PCT/JP2021/003380**

(87) International publication number:
**WO 2021/153779 (05.08.2021 Gazette 2021/31)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **31.01.2020  JP 2020015507
27.07.2020  JP 2020126672**

(71) Applicant: **Spiber Inc.
Tsuruoka-shi, Yamagata 997-0052 (JP)**

(72) Inventors:
• **CHUANG Yu Chun
Tsuruoka-shi Yamagata 997-0052 (JP)**
• **JIANG Li
Tsuruoka-shi Yamagata 997-0052 (JP)**
• **NAIDU Arin
Tsuruoka-shi Yamagata 997-0052 (JP)**

(74) Representative: **Handley, Matthew Edward et al
Venner Shipley LLP
200 Aldersgate
London EC1A 4HD (GB)**

Remarks:
The complete document including Reference Table(s) and the Sequence Listing(s) can be downloaded from the EPO website

(54) **MEAT SUBSTITUTE COMPOSITION**

(57) To provide a meat substitute composition having a texture and sensory properties closer to those of real meat than a meat substitute composition in the related art, the present disclosure provides meat substitute composition containing a polypeptide that satisfies the following (1) or (2):
(1) number of amino acid residues is 150 or more, an alanine residue content is 12 to 40%, and a glycine residue content is 11 to 55%; or
(2) an amino acid residue content of at least one type selected from the group consisting of serine, threonine, and tyrosine, an alanine residue content, and a glycine residue content account for 56% or more altogether.

*FIG. 1*

EXAMPLE 1
COMPARATIVE EXAMPLE 1
COMPARATIVE EXAMPLE 2

**Description**

Technical Field

The present invention relates to a meat substitute composition.

Background Art

In recent years, meat substitute compositions have been energetically developed to address food scarcity problems and to provide vegan food. In meat substitute compositions, the taste and mouthfeel of real meat are artificially replicated. Known examples of meat substitute compositions are cultured meat prepared by culturing cells collected from animals and meat alternatives using vegetable proteins from soybeans, peas, wheat, and the like (for example, Patent Literatures 1 to 3).

Citation List

Patent Literature

Patent Literature 1: JP 2014-520554 A
Patent Literature 2: JP 2017-517273 A
Patent Literature 3: JP 2009-537177 A

Summary of Invention

Technical Problem

At present, some meat alternatives using vegetable proteins have been commercialized. However, replication of the texture (such as appearance) and sensory properties (such as flavor and mouthfeel) of real meat remains to be solved.
An object of the invention is to provide a meat substitute composition having a texture and sensory properties closer to those of real meat than a meat substitute composition in the related art.

Solution to Problem

The present inventors have found that inclusion of a polypeptide that has a specific amino acid residue composition (such as structural protein) in a meat substitute composition in the related art using a vegetable protein enables replication of a texture and sensory properties closer to those of real meat. The invention is based on this finding.
That is, the invention relates to, for example, the following inventions. [1] A meat substitute composition containing a polypeptide that satisfies (1) or (2) below: (1) number of amino acid residues is 150 or more, an alanine residue content is 12% to 40%, and a glycine residue content is 11% to 55%; (2) an amino acid residue content of at least one type selected from the group consisting of serine, threonine, and tyrosine, an alanine residue content, and a glycine residue content account for 56% or more altogether. [2] The meat substitute composition according to [1], in which the polypeptide satisfies both (1) and (2). [3] The meat substitute composition according to [1] or [2], in which the polypeptide is a recombinant polypeptide. [4] The meat substitute composition according to any one of [1] to [3], in which the polypeptide has a plurality of repeating sequence units, and the repeating sequence units each have 6 to 200 amino acid residues. [5] The meat substitute composition according to any one of [1] to [4], in which the polypeptide contains an $(A)_n$ motif. [6] The meat substitute composition according to any one of [1] to [5], in which the polypeptide is a structural protein. [7] The meat substitute composition according to any one of [1] to [6], in which the polypeptide is fibroin. [8] The meat substitute composition according to any one of [1] to [7], in which the polypeptide is in the form of a fiber. [9] The meat substitute composition according to any one of [1] to [8], further containing a vegetable protein. [10] An artificial meat product including the meat substitute composition according to any one of [1] to [9] or a processed product of the meat substitute composition. [11] A method for making an artificial meat dish, the method involving cooking the meat substitute composition according to any one of [1] to [9] or the artificial meat product according to [10].

Advantageous Effects of Invention

According to the invention, a meat substitute composition is provided having a texture and sensory properties closer to those of real meat than a meat substitute composition in the related art.

Brief Description of Drawings

Fig. 1 is a photograph showing the appearance of hamburger patties using meat substitute compositions prepared in Test Example 1.
Fig. 2 is a photograph showing the appearance of hamburger patties using meat substitute compositions prepared in Test Example 1.
Figs. 3(a) to 3(c) are photographs showing the appearance of hamburger patties using meat substitute compositions prepared in Test Example 1.
Fig. 4 is a view illustrating an example of a spinning apparatus for producing polypeptide fibers.
Fig. 5 is a photograph showing the appearance of hamburger patties using meat substitute compositions prepared in Test Example 2.
Fig. 6 is a photograph showing the appearance of a hamburger patty using a meat substitute composition prepared in Test Example 2.
Fig. 7 is a graph illustrating changes in appearance (diameters of hamburger patties) that occur when cooking hamburger patties using meat or meat substitute compositions prepared in Test Example 3.
Fig. 8 is a schematic view illustrating data processing of a texture profile performed in Test Example 4.
Fig. 9 is a graph illustrating "hardness" of hamburger patties using meat or meat substitute compositions prepared in Test Example 4.
Fig. 10 is a graph illustrating "cohesiveness" of hamburger patties using meat or meat substitute compositions prepared in Test Example 4.
Fig. 11 is a graph illustrating "springiness" of hamburger patties using meat or meat substitute compositions prepared in Test Example 4.
Fig. 12 is a graph illustrating "chewiness" of hamburger patties using meat or meat substitute compositions prepared in Test Example 4.
Fig. 13 is a photograph showing the appearance of a hamburger patty using meat prepared in Test Example 5.
Fig. 14 is a photograph showing the appearance of hamburger patties using meat substitute compositions prepared in Test Example 5.
Fig. 15 is a graph illustrating changes in appearance (diameters of hamburger patties) that occur when cooking hamburger patties using meat or meat substitute compositions prepared in Test Example 6.

Description of Embodiments

Hereinafter, embodiments of the invention will be described in detail. However, the invention is not limited to the following embodiments.

[Meat Substitute Composition]

A meat substitute composition according to this embodiment contains a polypeptide having a specific amino acid residue composition.
Herein, the "meat substitute composition" represents a meat substitute that artificially replicates chemical properties (such as nutritional composition) or qualities (such as taste, flavor, mouthfeel, and appearance) of meat without depending on livestock-derived meat. What is called cultured meat and meat alternatives are also included in the meat substitute composition. The "cultured meat" that is produced in a laboratory by taking tissue or cells from an animal and culturing the cells is also called "lab-meat," "in vitro meat," or "clean meat." The "meat alternatives" using non-animal-derived raw materials such as plants are also called "imitation meat," "faux meat," "vegan meat," "plant meat," or "artificial meat."
The polypeptide according to this embodiment may satisfy either of the following (1) and (2): (1) the number of amino acid residues is 150 or more, the alanine residue content is 12% to 40%, and the glycine residue content is 11% to 55%; (2) the amino acid residue content of at least one type selected from the group consisting of serine, threonine, and tyrosine, the alanine residue content, and the glycine residue content account for 56% or more altogether.
Herein, the "alanine residue content" is a value represented by the following Formula: The alanine residue content = (the number of alanine residues contained in the polypeptide/the number of all amino acid residues in the polypeptide) $\times$ 100 (%).

With regard to the glycine residue content, the serine residue content, the threonine residue content, the tyrosine residue content, the glutamine residue content, and the lysine residue content, each value is calculated by the Formula, replacing "alanine residues" with glycine residues, serine residues, threonine residues, tyrosine residues, glutamine residues, and lysine residues, respectively.

The polypeptide that satisfies (1) may contain 150 or more amino acid residues. The number of amino acid residues may be, for example, 200 or more or 250 or more and is preferably 300 or more, 350 or more, 400 or more, 450 or more, or 500 or more.

The polypeptide that satisfies (1) may have an alanine residue content of 12% to 40%. The alanine residue content may be, for example, 15% to 40%, 18% to 40%, 20% to 40%, or 22% to 40%.

The polypeptide that satisfies (1) may have a glycine residue content of 11% to 55%. The glycine residue content may be, for example, 11% to 55%, 13% to 55%, 15% to 55%, 18% to 55%, 20% to 55%, 22% to 55%, or 25% to 55%.

The polypeptide that satisfies (1) contains a relatively large amount of alanine residues and glycine residues. Since the alanine residues and the glycine residues are amino acids having nonpolar side chains, the alanine residues and the glycine residues are arranged to face inward in a folding process during the polypeptide production and tend to have $\alpha$-helix structure or $\beta$-sheet structure. For this reason, the meat substitute composition containing the polypeptide that satisfies (1) is less likely to get mushy during and after cooking and have an increased chewy texture, which leads to improvement in texture (appearance) and sensory properties (mouthfeel). Furthermore, when the polypeptide that satisfies (1) is in the form of, for example, a fiber, sponge, gel, or film, secondary structures of these forms exhibit high strength and toughness, so that the above effects are exhibited more prominently.

In addition, glycine is said to have a detoxification effect to lower blood cholesterol and to remove harmful substances from the body. Alanine is said to promote alcohol metabolism by being converted into an energy source. Accordingly, the meat substitute composition according to this embodiment containing the polypeptide that satisfies (1) is expected to fulfill these nutritional functions.

In the polypeptide that satisfies (2), the amino acid residue content of at least one type selected from the group consisting of serine, threonine, and tyrosine (that is, any one of the serine residue content, the threonine residue content, the tyrosine residue content, the combined content of serine and threonine residues, the combined content of serine and tyrosine residues, the combined content of threonine and tyrosine residues, and the combined content of serine, threonine, and tyrosine residues), the alanine residue content, and the glycine residue content may account for 56% or more altogether (the total residue content may be 56% or more). The total residue content may be, for example, 57% or more, 58% or more, 59% or more, or 60% or more. The maximum of the total residue content is not particularly limited and may be, for example, 90% or less, 85% or less, or 80% or less.

In an embodiment, the sum of the serine residue content, the threonine residue content, and the tyrosine residue content in the polypeptide that satisfies (2) may be 4% or more, 4.5% or more, 5% or more, 5.5% or more, 6% or more, 6.5% or more, or 7% or more. The sum of the serine residue content, the threonine residue content, and the tyrosine residue content may be, for example, 35% or less, 33% or less, 30% or less, 25% or less, or 20% or less.

The polypeptide that satisfies (2) contains a relatively large amount of amino acid residues of one type selected from the group consisting of serine, threonine, and tyrosine and a relatively large amount of alanine residues and glycine residues. The serine residues, threonine residues, and tyrosine residues are relatively hydrophilic amino acid residues, and the alanine residues and glycine residues are relatively hydrophobic amino acid residues. For this reason, in the polypeptide that satisfies (2), the water-holding capacity and/or oil-absorption capacity of the polypeptide are optionally manipulated by changing the ratio between the amino acid residue content of one type selected from the group consisting of serine, threonine, and tyrosine and the alanine and glycine residue content. The polypeptide having the water-holding capacity and/or oil-absorption capacity has an increased affinity for aqueous ingredients and/or oily ingredients, which enhances the texture (appearance) and sensory properties (flavor and mouthfeel) of the meat substitute composition.

In addition, serine is said to have a detoxification effect to lower blood cholesterol and to remove harmful substances from the body. There is a report that tyrosine changes a neurotransmitter (dopa) and has preventive effects on dementia and Parkinson's disease. Accordingly, the meat substitute composition according to this embodiment containing the polypeptide that satisfies (2) is expected to fulfill these nutritional functions.

The polypeptide according to this embodiment preferably satisfies both (1) and (2) described above. Accordingly, the effects of the invention are exhibited more prominently.

In the polypeptide according to this embodiment, the serine residues, threonine residues, or tyrosine residues are distributed evenly, and the total residue content of serine residues, threonine residues, and tyrosine residues among optional 20 continuous amino acid residues may be 5% or more, 10% or more, 15% or more, 50% or less, 40% or less, 30% or less, or 20% or less.

The polypeptide according to an embodiment may have repeating sequences. That is, the polypeptide according to this embodiment may have a plurality of amino acid sequences (repeating sequence units) having high sequence identity in the polypeptide. The repeating sequence units are not particularly limited in amino acid sequences as long as the polypeptide as a whole satisfies (1) or (2) described above. The number of amino acid residues of the repeating sequence units is preferably 6 to 200. The sequence identity between the repeating sequence units may be, for example, 85% or more, 90% or more, 95% or more, 96% or more, 97% or more, 98% or more, or 99% or more.

The polypeptide according to an embodiment may contain glutamine residues and/or lysine residues. Containing glutamine residues or/and lysine residues, the polypeptide is crosslinked intramolecularly or intermolecularly in the presence of an edible crosslinking binder such as transglutaminase. The glutamine residue content may be, for example, 0% to 30%, 0% to 25%, 0% to 20%, 5% to 20%, 10% to 20%, or 15% to 20%. The lysine residue content may be, for example, 5% or more, 10% or more, 25% or less, 20% or less, 15% or less, or 10% or less.

The polypeptide according to an embodiment may be one containing an $(A)_n$ motif. Herein, the $(A)_n$ motif represents an amino acid sequence mainly containing alanine residues. The number of amino acid residues in the $(A)_n$ motif may be 2 to 27 and may be an integer of 2 to 20, 2 to 16, or 2 to 12. In addition, a proportion of the number of alanine residues to the total number of amino acid residues in the $(A)_n$ motif is 40% or more and may also be 60% or more, 70% or more, 80% or more, 83% or more, 85% or more, 86% or more, 90% or more, 95% or more, or 100% (which means that the $(A)_n$ motif consists of alanine residues).

In an embodiment, the $(A)_n$ motif may be contained in each of the repeating sequence units. The $(A)_n$ motif mainly contains alanine residues and tends to have $\alpha$-helix structure or $\beta$-sheet structure. Due to the $(A)_n$ motif contained in the repeating sequence units, the polypeptide according to this embodiment has these secondary structures in a repetitive manner, which prevents the meat substitute composition from getting mushy during and after cooking and increases the chewy texture. Accordingly, it is possible to enhance the texture (appearance) and sensory properties (mouthfeel) of the meat substitute composition. Furthermore, when the polypeptide is in the form of, for example, a fiber, sponge, gel, or film, secondary structures of these forms exhibit high strength and toughness, so that the above effects are exhibited more prominently.

The polypeptide according to this embodiment may be a structural protein. The structural protein represents a protein related to the structure of a living body, a protein included in a structure created by a living body, or a protein derived from those proteins. Examples of the structural protein include fibroin, keratin, collagen, elastin, and resilin.

The structural protein is not particularly limited as long as it satisfies (1) or (2) described above, but fibroin is preferable. Examples of the fibroin include silk fibroin, spider silk fibroin, hornet silk fibroin, and proteins derived from these kinds of fibroin.

An example of the fibroin includes naturally derived fibroin. An example of the naturally derived fibroin includes fibroin produced by insects or spiders.

Examples of the fibroin produced by insects include silk fibroin produced by silkworms such as Bombyx mori, Bombyx mandarina, Antheraea yamamai, Anteraea pernyi, Eriogyna pyretorum, Philosamia cynthia ricini, Samia cynthia, Caligura japonica, Antheraea mylitta, and Antheraea assama and hornet silk fibroin secreted by larvae of Vesp simillima xanthoptera.

More specific examples of the fibroin produced by insects include silkworm fibroin L chain (GenBank Accession Numbers M76430 (base sequence) and AAA27840.1 (amino acid sequence)).

Examples of the fibroin produced by spiders include spider silk proteins produced by spiders belonging to the order Araneae. More specific examples include spider silk fibroin produced by spiders belonging to the genus Araneus such as Araneus ventricosus, Araneus diadematus, Araneus pinguis, Araneus pentagrammicus, and Araneus nojimai, spiders belonging to the genus Neoscona such as Neoscona scylla, Neoscona nautica, Neoscona adianta, and Neoscona scylloides, spiders belonging to the genus Pronus such as Pronous minutus, spiders belonging to the genus Cyrtarachne such as Cyrtarachne bufo and Cyrtarachne inaequalis, spiders belonging to the genus Gasteracantha such as Gasteracantha kuhlii and Gasteracantha mammosa, spiders belonging to the genus Ordgarius such as Ordgarius hobsoni and Ordgarius sexspinosus, spiders belonging to the genus Argiope such as Argiope amoena, Argiope minuta, and Argiope bruennichi, spiders belonging to the genus Arachnura such as Arachnura logio, spiders belonging to the genus Acusilas such as Acusilas coccineus, spiders belonging to the genus Cytophora such as Cyrtophora moluccensis, Cyrtophora exanthematica, and Cyrtophora unicolor, spiders belonging to the genus Poltys such as Poltys illepidus, spiders belonging to the genus Cyclosa such as Cyclosa octotuberculata, Cyclosa sedeculata, Cyclosa vallata, and Cyclosa atrata, and spiders belonging to the genus Chorizopes such as Chorizopes nipponicus, and spider silk proteins produced by spiders belonging to the family Tetragnathidae such as spiders belonging to the genus Tetragnatha such as Tetragnatha praedonia, Tetragnatha maxillosa, Tetragnatha extensa, and Tetragnatha squamata, spiders belonging to the genus Leucauge such as Leucauge magnifica, Leucauge blanda, and Leucauge subblanda, spiders belonging to the genus Nephila such as Nephila clavata and Nephila pilipes, spiders belonging to the genus Menosira such as Menosira ornata, spiders belonging to the genus Dyschiriognatha such as Dyschiriognatha tenera, spiders belonging to the genus Latrodectus such as Latrodectus mactans, Latrodectus hasseltii, Latrodectus geometricus, and Latrodectus tredecimguttatus, and spiders belonging to the genus Euprosthenops. Examples of the spider silk fibroin include dragline silk proteins such as MaSps (MaSp1 and MaSp2) and ADFs (ADF3 and ADF4), MiSps (MiSp1 and MiSp2), AcSp, PySp, and Flag.

More specific examples of the spider silk fibroin produced by spiders include fibroin-3 (adf-3) [derived from Araneus diadematus] (GenBank Accession Number AAC47010 (amino acid sequence), U47855 (base sequence)), fibroin-4 (adf-4) [derived from Araneus diadematus] (GenBank Accession Number AAC47011 (amino acid sequence), U47856 (base sequence)), dragline silk protein spidroin 1 [derived from Nephila clavipes] (GenBank Accession Number AAC04504 (amino acid sequence), U37520 (base sequence)), major ampullate spidroin 1 [derived from Latrodectus hesperus] (GenBank Accession Number ABR68856 (amino acid sequence), EF595246 (base sequence)), dragline silk protein spidroin 2 [derived from Nephila clavata] (GenBank Accession Number AAL32472 (amino acid sequence), AF441245 (base sequence)), major ampullate spidroin 1 [derived from Euprosthenops australis] (GenBank Accession Number CAJ00428 (amino acid sequence), AJ973155 (base sequence)), major ampullate spidroin 2 [derived from Euprosthenops australis] (GenBank Accession Number CAM32249.1 (amino acid sequence), AM490169 (base sequence)), minor ampullate silk protein 1 [derived from Nephila clavipes] (GenBank Accession Number AAC14589.1 (amino acid sequence)), minor ampullate silk protein 2 [derived from Nephila clavipes] (GenBank Accession Number AAC14591.1 (amino acid sequence)), and minor ampullate spidroin-like protein [derived from Nephilengys cruentata] (GenBank Accession Number ABR37278.1 (amino acid sequence)).

A more specific example of the naturally derived fibroin includes fibroin having sequence information registered in NCBI GenBank. Among sequence information registered in NCBI GenBank, for example, the sequence information of the fibroin is checked by extracting sequences with a keyword of spidroin, ampullate, fibroin, "silk and polypeptide," or "silk and protein" in the DEFINITION from sequences containing INV as DIVISION, by extracting sequences having a specific product character string from CDS, and by extracting sequences having a character-string specific to TISSUE TYPE from SOURCE.

The fibroin may be modified fibroin. The modified fibroin herein represents artificially produced fibroin (artificial fibroin). The modified fibroin may be fibroin having a domain sequence different from or identical to an amino acid sequence of naturally derived fibroin. The modified fibroin is a protein having a domain sequence represented by Formula 1: $[(A)_n$ motif-REP$]_m$ or Formula 2: $[(A)_n$ motif-REP$]_m$-$(A)_n$ motif. Furthermore, an amino acid sequence (N-terminal sequence and C-terminal sequence) may be added to either or both of the N-terminus and the C-terminus of the domain sequence of the modified fibroin. The N-terminal sequence and the C-terminal sequence are typically, but are not limited to, regions not having repeats of fibroin-specific amino acid motifs and having about 100 amino acid residues.

The modified fibroin may be one obtained using an amino acid sequence of naturally derived fibroin as it is or one obtained by modifying an amino acid sequence of naturally derived fibroin (such as fibroin having a modified amino acid sequence obtained by modifying a cloned gene sequence of naturally derived fibroin) or one obtained by artificially designed and synthesized independently of naturally derived fibroin (such as fibroin having a desired amino acid sequence obtained by chemically synthesizing a nucleic acid that encodes the designed amino acid sequence).

Herein, the term "domain sequence" is an amino acid sequence that generates a crystalline region (typically corresponding to $(A)_n$ motif of an amino acid sequence) and an amorphous region (typically corresponding to REP of an amino acid sequence) specific to fibroin and is represented by Formula 1: $[(A)_n$ motif-REP$]_m$ or Formula 2: $[(A)_n$ motif-REP$]_m$-$(A)_n$ motif. Here, the $(A)_n$ motif is an amino acid sequence mainly containing alanine residues, and the number of amino acid residues is 2 to 27. The number of amino acid residues in the $(A)_n$ motif may be 2 to 20, 2 to 16, or 2 to 12 or may be 4 to 27, 4 to 20, 8 to 20, 10 to 20, 4 to 16, 8 to 16, or 10 to 16. In addition, a proportion of the number of alanine residues to the total number of amino acid residues in the $(A)_n$ motif is 40% or more and may also be 60% or more, 70% or more, 80% or more, 83% or more, 85% or more, 86% or more, 90% or more, 95% or more, or 100% (which means that the $(A)_n$ motif consists of alanine residues). At least seven of a plurality of $(A)_n$ motifs in the domain sequence may consist of alanine residues. The "REP" is an amino acid sequence consisting of 2 to 200 amino acid residues. The "REP" may also be an amino acid sequence consisting of 10 to 200 amino acid residues. The symbol "m" represents an integer from 2 to 300 and may be an integer from 10 to 300. The plurality of $(A)_n$ motifs may be identical or different amino acid sequences. A plurality of REPs may be identical or different amino acid sequences. In each REP, the sum of the serine residue content, the threonine residue content, and the tyrosine residue content may be 5% or more, 6% or more, 7% or more, 8% or more, 9% or more, 10% or more, 11% or more, 12% or more, 13% or more, 14% or more, 15% or more, 50% or less, 40% or less, 30% or less, or 20% or less. Furthermore, in all REPs, the sum of the serine residue content, the threonine residue content, and the tyrosine residue content (the sum of the serine residue content, the threonine residue content, and the tyrosine residue content when all REPs are regarded as one polypeptide) may be 5% or more, 6% or more, 7% or more, 8% or more, 9% or more, 10% or more, 11% or more, 12% or more, 13% or more, 14% or more, 15% or more, 50% or less, 40% or less, 30% or less, or 20% or less. In all REPs, the glutamine residue content may be 5% or more, 6% or more, 7% or more, 8% or more, 9% or more, 10% or more, 11% or more, 12% or more, 13% or more, 14% or more, 15% or more, 30% or less, or 20% or less. The number of amino acid residues in the domain sequence may be, for example, 6 to 300, 6 to 250, or 6 to 200.

The modified fibroin according to this embodiment is obtained by, for example, modification of an amino acid sequence corresponding to substitution, deletion, insertion, and/or addition of one or a plurality of amino acid residues in a cloned gene sequence of naturally derived fibroin. The amino acid residues are substituted, deleted, inserted, and/or added by methods well known to those skilled in the art such as site-directed mutagenesis. Specifically, the substitution, deletion, insertion, and/or addition of amino acid residues are performed according to the methods recited in Nucleic Acid Res. 10, 6487 (1982) and Methods in Enzymology, 100, 448 (1983).

The fibroin is not particularly limited as long as it satisfies (1) or (2) described above. A specific example of the fibroin includes fibroin (modified fibroin) shown in the following Table 1.

[Table 1]

| | Alanine residue content (%) | Glycine residue content (%) | Serine residue content (%) | Threonine residue content (%) | Tyrosine residue content (%) | Glutamine residue content (%) | Lysine residue content (%) |
|---|---|---|---|---|---|---|---|
| PRT799 (SEQ ID NO: 1) | 19.8 | 31.0 | 9.3 | 0 | 7.0 | 17.3 | 0.1 |
| PRT966 (SEQ ID NO: 2) | 19.7 | 31.2 | 9.4 | 0 | 7.1 | 0 | 0 |
| PRT918 (SEQ ID NO: 3) | 19.5 | 30.9 | 9.7 | 0 | 7.0 | 0 | 0 |
| PRT313 (SEQ ID NO: 4) | 25.7 | 36.0 | 8.9 | 0 | 6.4 | 8.2 | 0 |
| PRT1181 (SEQ ID NO: 5) | 25.7 | 28.6 | 8.9 | 0 | 6.4 | 0 | 0 |
| PRT1010 (SEQ ID NO: 6) | 18.4 | 29.2 | 9.1 | 0 | 6.6 | 0 | 5.5 |

The polypeptide according to an embodiment may be purified. Preferably, the purified polypeptide consists of the polypeptide but may contain impurities that are inevitably mixed.

The polypeptide according to an embodiment may be a recombinant polypeptide. The recombinant polypeptide represents a polypeptide produced by genetic recombination. The recombinant polypeptide may be isolated from a non-animal-derived genetically modified organism depending on, for example, the use of the meat substitute composition.

The polypeptide according to an embodiment may be derived from a non-plant source. Specifically, the polypeptide may be, for example, a polypeptide purified from a non-plant or a polypeptide produced from a gene isolated from a non-plant using genetic recombination.

The polypeptide according to an embodiment may be antimicrobial. Specifically, for example, the polypeptide may have an antimicrobial amino acid sequence or may have an antimicrobial protein motif. Compared with a meat substitute composition in the related art, a meat substitute composition containing such a polypeptide has a long shelf life and allows storage or transportation particularly at room temperature.

The polypeptide according to this embodiment may have a shape (in the form of, for example, fiber, gel, film, porous body (sponge), particle, or molded body) containing or consisting of, for example, the polypeptide. The polypeptide according to this embodiment is preferably in the form of a fiber (polypeptide fiber) so that it is possible to exhibit the effects of the invention, that is, a texture and sensory properties closer to those of real meat, more prominently than a meat substitute composition in the related art.

The polypeptide fiber may be a long fiber or short fiber. The short fiber may have a length of, for example, 1 to 20 mm, 1 to 15 mm, 1 to 10 mm, or 1 to 5 mm.

The polypeptide fiber may be a filament yarn (such as multifilament and monofilament), spun yarn, twisted yarn, false twisted yarn, textured yarn, combined filament yarn, or blended yarn.

The polypeptide fiber is produced by a known spinning method. That is, for example, first, the polypeptide according to this embodiment is added to and dissolved in a solvent or solution such as dimethyl sulfoxide (DMSO), N,N-dimethylformamide (DMF), hydrochloric acid, formic acid, or hexafluoroisopropanol (HFIP) together with an inorganic salt serving as a dissolution promoter to prepare a dope solution. Next, this dope solution is used for implementing a known spinning method such as wet spinning, dry spinning, dry-wet spinning, or melt spinning, thereby obtaining the target polypeptide fiber.

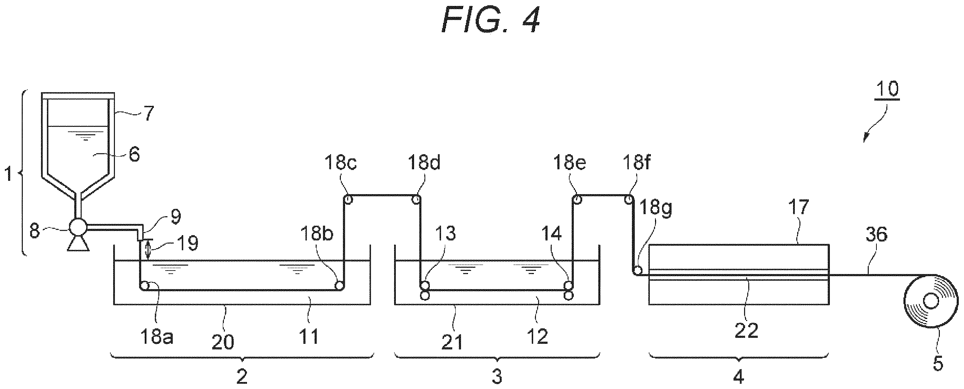

Fig. 4 is a view illustrating an example of a spinning apparatus for producing polypeptide fibers. A spinning apparatus 10 illustrated in Fig. 4 is an example of a spinning apparatus for dry-wet spinning and includes an extruder 1, an undrawn yarn-producing device 2, a wet heat drawing device 3, and a drying device 4.

A spinning method using the spinning apparatus 10 will now be described. First, a dope solution 6 stored in a reservoir 7 is extruded from a spinneret 9 by a gear pump 8. Next, the extruded dope solution 6 is fed into a coagulation liquid 11 in a coagulation liquid tank 20 through an air gap 19, and a solvent is removed, whereby polypeptide is coagulated to form a fibrous coagulated body. Next, the fibrous coagulated body is fed into hot water 12 in a drawing bath 21 and drawn. A draw ratio is determined by a speed ratio between a feed nip roller 13 and a take-up nip roller 14. After that, the drawn fibrous coagulated body is fed to the drying device 4 and dried in a yarn path 22, and a polypeptide fiber 36 is obtained as a yarn package 5. Reference numerals 18a to 18g represent yarn guides.

The coagulation liquid 11 may be any solvent or solution that enables desolvation, and examples of the coagulation liquid 11 include $C_{1-5}$ lower alcohols such as methanol, ethanol, and 2-propanol and also include acetone, sodium hydroxide, sodium carbonate, and sodium hydrogen carbonate. The coagulation liquid 11 may contain water appropriately. The coagulation liquid 11 preferably has a temperature of 0°C to 30°C. In a case where a syringe pump having a nozzle with a diameter of 0.1 to 0.6 mm is used as the spinneret 9, an extrusion speed is preferably 0.2 to 6.0 ml/h, and more preferably 1.4 to 4.0 ml/h per hole. The coagulated polypeptide travels a distance of, for example, 200 to 500 mm in the coagulation liquid 11 (substantially, a distance from the yarn guide 18a to the yarn guide 18b), but the distance is not limited as long as desolvation is efficiently performed. A take-up speed of undrawn yarn may be, for example, 1 to 20 m/min and is preferably 1 to 3 m/min. A residence time in the coagulation liquid 11 may be, for example, 0.01 to 3 minutes and is preferably 0.05 to 0.15 minutes. Furthermore, the drawing (pre-drawing) may be performed in the coagulation liquid 11. The coagulation liquid tank 20 may be provided with multiple stages, and the drawing may be performed in each stage or in a specific stage as occasion demands.

In addition to pre-drawing in the coagulation liquid tank 20 and wet-heat drawing in the drawing bath 21, for example, dry-heat drawing is also employed to obtain polypeptide fibers.

The wet-heat drawing is performed in hot water, in a solution obtained by adding an organic solvent or the like to hot water, or with heated steam. The temperature may be, for example, 50 to 90°C and preferably 75 to 85°C. In the wet-heat drawing, the undrawn yarn (or pre-drawn yarn) is drawn to, for example, 1 to 10 times and preferably 2 to 8 times its original size.

The dry-heat drawing is performed using, for example, an electric tubular furnace and a dry heat plate. The temperature may be, for example, 140 to 270°C and is preferably 160 to 230°C. In the dry-heat drawing, the undrawn yarn (or pre-drawn yarn) is drawn to, for example, 0.5 to 8 times and preferably 1 to 4 times its original size.

The wet-heat drawing and the dry-heat drawing may be performed independently, or performed through multiple stages, or in combination. In other words, the wet-heat drawing and the dry-heat drawing may be appropriately combined: for example, the wet-heat drawing is performed in the first stage and the dry-heat drawing in the second stage, or the wet-heat drawing is performed in the first and second stages and the dry-heat drawing in the third stage.

The minimum of the final draw ratio is preferably any one of over 1 time, 2 times or more, 3 times or more, 4 times or more, 5 times or more, 6 times or more, 7 times or more, 8 times or more, and 9 times or more the undrawn yarn (or pre-drawn yarn), and the maximum is preferably 40 times or less, 30 times or less, 20 times or less, 15 times or less, 14 times or less, 13 times or less, 12 times or less, 11 times or less, or 10 times or less the undrawn yarn (or pre-drawn yarn). The drawn yarn exhibits higher strength, which achieves the effect of the invention more prominently.

The polypeptide fibers according to this embodiment may have stress of 0.5 gf/d or more. The stress is preferably 0.8 gf/d or more, and more preferably 1 gf/d or more. The stress falling inside this range achieves the effect of further increasing the production yield of the meat substitute composition. The strength is a value determined by a standard tensile test of a multifilament yarn.

The polypeptide fibers according to this embodiment may have a diameter less than 50 μm. Preferably, the protein fibers have a diameter of, for example, less than 45 μm or less than 40 μm, more preferably 35 μm or less, 32 μm or less, or 30 μm or less, and still more preferably, 15 μm or less or 10 μm or less. Using protein fibers having a smaller diameter excellently increases verisimilitude, which provides a mouthfeel and a texture closer to those of real meat. The protein fibers may have a diameter of, for example, 5 μm or more, 10 μm or more, 15 μm or more, 18 μm or more, 20 μm or more, 22 μm or more, 25 μm or more, 30 μm or more, 32 μm or more, 35 μm or more, 40 μm or more, or 45 μm or more. The protein fibers may have a diameter of, for example, 5 to 50 μm, 10 to 50 μm, 15 to 50 μm, 5 to 45 μm, 5 to 40 μm, 5 to 35 μm, 5 to 32 μm, 5 to 50 μm, 10 to 45 μm, 10 to 40 μm, 10 to 35 μm, 10 to 32 μm, or 10 to 30 μm.

The polypeptide according to this embodiment may be in the form of a molded body (polypeptide resin). Using the polypeptide molded body makes it possible to replicate real "meat on the bone."

The meat substitute composition according to this embodiment may contain the polypeptide of one type or two or more types.

The meat substitute composition according to this embodiment may contain protein components other than the polypeptide. The protein components are preferably those derived from non-animal sources used in a meat substitute composition in the related art, specifically, proteins derived from soybean, pea, wheat, oat, rye, barley, canola, sunflower, sorghum, rice, amaranth, potato, tapioca, arrowroot, canna, lupine, rapeseed, algae, edible filamentous fungus, and mixtures thereof.

The polypeptide content in the meat substitute composition according to this embodiment may be, for example, 0.1 wt% or more, 0.2 wt% or more, 0.3 wt% or more, 0.4 wt% or more, 0.5 wt% or more, 0.6 wt% or more, 0.7 wt% or more, 0.8 wt% or more, 0.9 wt% or more, 1 wt% or more, 1.5 wt% or more, 2 wt% or more, 2.5 wt% or more, 3 wt% or more, 3.5 wt% or more, 4 wt% or more, 4.5 wt% or more, 5 wt% or more, 10 wt% or more, 15 wt% or more, 20 wt% or more, 30 wt% or more, 40 wt% or more, or 50 wt% or more based on the total amount of the meat substitute composition. The polypeptide content in the meat substitute composition according to this embodiment may be, for example, 90 wt% or less, 80 wt% or less, 70 wt% or less, 60 wt% or less, 50 wt% or less, 40 wt% or less, 30 wt% or less, 20 wt% or less, 15 wt% or less, 10 wt% or less, 5 wt% or less, 4.5 wt% or less, 4 wt% or less, 3.5 wt% or less, 3 wt% or less, 2.5 wt% or less, 2 wt% or less, 1.5 wt% or less, 1 wt% or less, 0.9 wt% or less, 0.8 wt% or less, 0.7 wt% or less, 0.6 wt% or less, 0.5 wt% or less, 0.4 wt% or less, 0.3 wt% or less, or 0.2 wt% or less based on the total amount of the meat substitute composition. The polypeptide content in the meat substitute composition according to this embodiment may be, for example, 0.1 to 10 wt%, 0.2 to 10 wt%, 0.3 to 10 wt%, 0.4 to 10 wt%, 0.5 to 10 wt%, 0.6 to 10 wt%, 0.7 to 10 wt%, 0.8 to 10 wt%, 0.9 to 10 wt%, 1 to 10 wt%, 0.1 to 9 wt%, 0.1 to 8 wt%, 0.1 to 7 wt%, 0.1 to 6 wt%, 0.1 to 5 wt%, 0.5 to 9 wt%, 0.5 to 8 wt%, 0.5 to 7 wt%, 0.5 to 6 wt%, 0.5 to 5 wt%, 1 to 9 wt%, 1 to 8 wt%, 1 to 7 wt%, 1 to 6 wt%, 1 to 5 wt%, 1 to 4 wt%, 1.5 to 10 wt%, 1.5 to 9 wt%, 1.5 to 8 wt%, 1.5 to 7 wt%, 1.5 to 6 wt%, 1.5 to 5 wt%, 2 to 10 wt%, 2 to 9 wt%, 2 to 8 wt%, 2 to 7 wt%, 2 to 6 wt%, 2 to 5 wt%, 3 to 10 wt%, 3 to 9 wt%, 3 to 8 wt%, 3 to 7 wt%, 3 to 6 wt%, or 3 to 5 wt% based on the total amount of the meat substitute composition.

The polypeptide content in the meat substitute composition according to this embodiment may be, for example, 0.1 vol% or more, 0.2 vol% or more, 0.3 vol% or more, 0.4 vol% or more, 0.5 vol% or more, 0.6 vol% or more, 0.7 vol% or more, 0.8 vol% or more, 0.9 vol% or more, 1 vol% or more, 1.5 vol% or more, 2 vol% or more, 2.5 vol% or more, 3 vol% or more, 3.5 vol% or more, 4 vol% or more, 4.5 vol% or more, 5 vol% or more, 10 vol% or more, 15 vol% or more, 20 vol% or more, 30 vol% or more, 40 vol% or more, or 50 vol% or more based on the total amount of the meat substitute composition. The polypeptide content in the meat substitute composition according to this embodiment may be, for example, 90 vol% or less, 80 vol% or less, 70 vol% or less, 60 vol% or less, 50 vol% or less, 40 vol% or less, 30 vol% or less, 20 vol% or less, 15 vol% or less, 10 vol% or less, 5 vol% or less, 4.5 vol% or less, 4 vol% or less, 3.5 vol% or less, 3 vol% or less, 2.5 vol% or less, 2 vol% or less, 1.5 vol% or less, 1 vol% or less, or 0.5 vol% or less based on the total amount of the meat substitute composition. The polypeptide content in the meat substitute composition according to this embodiment may be, for example, 0.1 to 10 vol%, 0.5 to 10 vol%, 1 to 10 vol%, 1.5 to 10 vol%, 2.5 to 10 vol%, 0.1 to 5 vol%, 0.1 to 4 vol%, 0.1 to 3 vol%, 0.1 to 2.5 vol%, 5 to 10 vol%, 0.5 to 8 vol%, 0.5 to 5 vol%, 0.5 to 4 vol%, 0.5 to 3.5 vol%, 0.5 to 3 vol%, 1 to 8 vol%, 1 to 5 vol%, 1.5 to 5 vol%, 2.5 to 5 vol%, 3 to 8 vol%, or 0.5 to 2.5 vol% based on the total amount of the meat substitute composition.

The polypeptide content in the meat substitute composition according to this embodiment may be, for example, 10 wt% or more, 20 wt% or more, 30 wt% or more, 40 wt% or more, or 50 wt% or more based on the sum of protein components contained in the meat substitute composition. The polypeptide content in the meat substitute composition according to this embodiment may be, for example, 90 wt% or less, 80% wt or less, 70 wt% or less, 60 wt% or less, 50 wt% or less, 40 wt% or less, 30 wt% or less, or 20 wt% or less based on the sum of protein components contained in the meat substitute composition.

The protein component content in the meat substitute composition according to this embodiment may be, for example, 0.5 wt% or more, 1 wt% or more, 2 wt% or more, 3 wt% or more, 4 wt% or more, 5 wt% or more, 6 wt% or more, 7 wt% or more, 8 wt% or more, 9 wt% or more, 10 wt% or more, 20 wt% or more, 30 wt% or more, 40 wt% or more, or 50 wt% or more based on the total amount of the meat substitute composition. The protein component content in the meat substitute composition according to this embodiment may be, for example, 90 wt% or less, 80% wt or less, 70 wt% or less, 60 wt% or less, 50 wt% or less, 40 wt% or less, 30 wt% or less, 20 wt% or less, or 10 wt% or less based on the total amount of the meat substitute composition.

The meat substitute composition according to this embodiment may contain, for example, water, vegetable oil, carbohydrates, salts, minerals, colorants, antioxidants, thickening stabilizers, dietary fibers, flavorings, and edible crosslinking binders.

Examples of the vegetable oil include corn oil, olive oil, soybean oil, peanut oil, almond oil, sesame oil, cottonseed oil, rapeseed oil, canola oil, safflower oil, sunflower oil, linseed oil, palm oil, walnut oil, algal oil, coconut oil, shea butter, mango butter, cocoa butter, wheat germ oil, rice bran oil, or oil produced by bacteria, algae, archaea, or fungi or produced by genetically modified bacteria, algae, archaea, or fungi. Examples of the carbohydrates include oligosaccharides, sugars, and plant-derived starches such as arrowroot, corn starch, dogtooth violet starch (katakuri starch), potato starch, sago, and tapioca. Examples of the salts include sodium chloride, potassium chloride, glutamate (for example, monosodium glutamate), glycine salt, guanylate, inosinate, and 5'-ribonucleotide salt. Examples of the minerals include salts of aluminum, ammonium, calcium, magnesium, and potassium. The antioxidants may be natural or synthetic, and examples of the antioxidants include those capable of preventing discoloration due to oxidation of colored vegetable proteins. Examples of the thickening stabilizers include alginic acid and a salt thereof, agar, carrageenan and a salt thereof, processed eucheuma algae, gum (carob bean, guar, tragacanth, and xanthan), pectin, and sodium carboxymethyl cellulose. Examples of the dietary fibers include soy fibers and gluten filaments. Examples of the flavorings include spice extract, spice oil, natural fumigation liquid, natural fumigation extract, yeast extract, shiitake extract, and flavoring agents such as onion flavor, garlic flavor, and herb flavor. Examples of the edible crosslinking binders include those capable of promoting filament formation, specifically, konjac glucomannan (KGM) powder, edible crosslinking binders such as transglutaminase, β-glucan such as Pureglucan (Registered trademark, available from Takeda), calcium salt, and magnesium salt.

The meat substitute composition according to this embodiment is obtained according to an ordinary method except that the polypeptide according to the invention is added thereto. The meat substitute composition according to this embodiment is obtained by, for example, uniformly mixing the polypeptide according to the invention with the foregoing various ingredients as necessary and forming the mixture into a desired shape. Alternatively, the meat substitute composition according to this embodiment is obtained by, for example, uniformly mixing the various ingredients other than the polypeptide according to the invention, and then adding the polypeptide according to the invention to the mixture, followed by uniformly mixing the mixture and forming the mixture into a desired shape. Furthermore, in a case where the polypeptide according to the invention is in the form of a fiber, the meat substitute composition according to this embodiment is obtained by, for example, uniformly mixing the various ingredients other than the polypeptide according to the invention, and then forming the mixture into a desired shape through treatment such as stitching, weaving, braiding, knitting, and needle punching.

The invention also relates to an artificial meat product including the meat substitute composition according to the invention or a processed product thereof. Examples of the processed product of the meat substitute composition include a heated meat substitute composition, a seasoned meat substitute composition, and a meat substitute composition cooked with other ingredients (including cooked and half-cooked products) as necessary. The meat substitute composition may be processed into a variety of foods for either humans or animals. For example, an end product may be a human-edible meat substitute composition imitating the qualities of a minced meat product, steak, sirloin tip, kebab, shredded meat product, sliced meat product, or nugget. These products may be placed on a tray, and then packed, vacuum-packed, placed in a retortable can or pouch, or frozen.

The invention also relates to a method for making an artificial meat dish, the method involving cooking the meat substitute composition according to the invention or the artificial meat product. The meat substitute composition or the artificial meat product is cooked according to an ordinary method.

Examples

Hereinafter, the invention will be described in more detail based on Test Examples. However, the invention is not limited to the following Test Examples.

[Test Example 1: Preparation and Evaluation of Meat Substitute Composition]

<Comparative Example 1>

Each ingredient listed in Table 2 was added to commercially available textured vegetable protein (TVP, available from Bob's Red Mill) at a rate of weight-percent (percentage based on the total weight of the finally obtained meat substitute composition of Comparative Example 1) shown in Table 2, and the ingredients were mixed uniformly. The mixture was heated at 700 W for two minutes in a household microwave oven. The resultant was left to cool to room temperature, and then water was added in an amount equal to evaporated liquid (7 g).

[Table 2]

| Ingredient | Manufacturer | Weight-percent based on the total weight (%) |
| --- | --- | --- |
| Water | - | 33.5% |
| Beet juice | Biotta | 13.4% |
| Beef consommé | Edward & Sons | 1.6% |

(continued)

| Ingredient | Manufacturer | Weight-percent based on the total weight (%) |
|---|---|---|
| Yeast flakes | KAL | 2.1% |
| Superfine sugar | Nippon Beet Sugar Manufacturing Co., Ltd. | 2.1% |
| Trace mineral drops (Iron) | Trace Minerals Research | 1.1% |
| Salt | Ajinomoto Co., Inc. | 1.1% |

Next, each ingredient listed in Table 3 was added to the obtained mixture at a rate of weight-percent (percentage based on the total weight of the finally obtained meat substitute composition of Comparative Example 1) shown in Table 3, and the ingredients were mixed uniformly.

[Table 3]

| Ingredient | Manufacturer | Weight percent based on the total weight (%) |
|---|---|---|
| Dogtooth violet starch (katakuriko) | Nisshin Seifun Group Inc. | 2.1% |
| Potato protein isolate | Xi-an Sheerherb Biological Technology | 2.1% |
| Soy protein isolate | Now Foods | 1.1% |

Each ingredient listed in Table 4 was added to the obtained mixture at a rate of weight-percent (percentage based on the total weight of the finally obtained meat substitute composition of Comparative Example 1) shown in Table 4, and the ingredients were mixed uniformly. Through this process, a meat substitute composition of Comparative Example 1 was obtained.

[Table 4]

| Ingredient | Manufacturer | Weight percent based on the total weight (%) |
|---|---|---|
| Salad oil | The Nisshin OilliO Group, Ltd. | 2.4% |
| Olive oil | The Nisshin OilliO Group, Ltd. | 3.2% |
| Fiber cellulose powder | Nutricology | 2.1% |
| Coconut oil | California Gold Nutrition | 7.5% |

Sixty grams of the meat substitute composition of Comparative Example 1 was picked up, shaped into a hamburger patty, and cooked in a frying pan, followed by sensory evaluation.

<Comparative Example 2>

About 3 g of salad oil was mixed into 60 g of the meat substitute composition of Comparative Example 1, thereby obtaining a meat substitute composition of Comparative Example 2. This meat substitute composition was picked up, shaped into a hamburger patty, and cooked in a frying pan, followed by sensory evaluation.

<Example 1>

Using a mini warper (SW550, available from CCI TECH INC.), several hundreds of long fibers having a length of 3.6 m were obtained from a silk bobbin (derived from silkworm). The obtained long fibers were cut with a powerful desktop fiber-cutting machine (NP-300, available from INTEC CO. LTD.) to obtain short silk fibers (about 100 g) having a length of 3 mm.

About 3 g of the short silk fibers were mixed into 60 g of the meat substitute composition of Comparative Example 1, thereby obtaining a meat substitute composition of Example 1. This meat substitute composition was picked up, shaped into a hamburger patty, and cooked in a frying pan, followed by sensory evaluation.

<Example 2>

About 3 g of the short silk fibers obtained in Example 1 were immersed in hot water at a temperature over 80°C for 1 minute, followed by filtration. After the filtration, about 10 g of salad oil was added to the short silk fibers and dispersed uniformly. A dispersion liquid containing the obtained short silk fibers was mixed into 60 g of the meat substitute composition of Comparative Example 1, thereby obtaining a meat substitute composition of Example 2. This meat substitute composition was picked up, shaped into a hamburger patty, and cooked in a frying pan, followed by sensory evaluation.

(Sensory Evaluation)

The meat substitute compositions of Comparative Examples 1 and 2 and Examples 1 and 2 were cooked to obtain hamburger patties, and sensory evaluation was performed on each hamburger patty to rate appearance, palatability, aroma (smell), and mouthfeel. The sensory evaluation was performed by one panel, and each property was rated on a scale of 1 to 4 (1: poor, 2: fair, 3: average, 4: good). Results are shown in Table 5.

[Table 5]

| | Appearance | Palatability | Aroma | Mouthfeel | Comments (Advantages) | Comments (Disadvantages) |
|---|---|---|---|---|---|---|
| Comparative Example 1 | 1 | 1 | 1 | 1 | *Nothing in particular. | *Mushy, a shapeless appearance. *A dry and crumbly mouthfeel. *No meaty quality. |
| Comparative Example 2 | 1 | 1 | 1 | 2 | *Salad oil made the hamburger patty juicy. The dry and crumbly texture in Comparative Example 1 was slightly improved. *Addition of oil provided a taste and a smell closer to those of meat. | *Although some properties were improved, the dry and crumbly texture remained to be solved. *No tenderness like real meat. |
| Example 1 | 3 | 3 | 2 | 3 | *Fibers played a role of connective tissues and settled the hamburger patty, which caused a mouthfeel closer to that of real meat. *Fibers were well dispersed and did not provide "fibrous" feeling in the mouth. | *White fibers stood out, which resulted in a poor appearance. |

(continued)

|  | Appearance | Palatability | Aroma | Mouthfeel | Comments (Advantages) | Comments (Disadvantages) |
|---|---|---|---|---|---|---|
| Example 2 | 4 | 4 | 2 | 4 | *Fibers played a role of connective tissues and settled the hamburger patty further, which caused an appearance and a mouthfeel much closer to those of real meat. *Fibers absorbed oil, which provided the hamburger patty with tenderness as a whole. *The smell of oil provided a meaty quality. | *Some fibers were too tough to chew because of clumping together. |

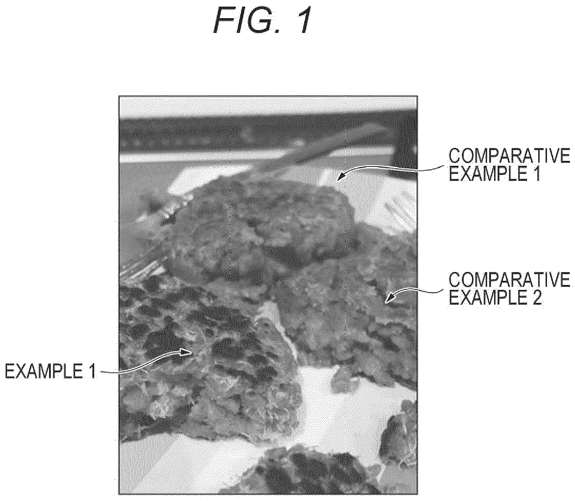

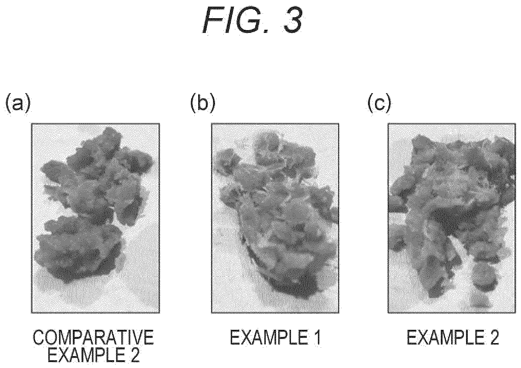

Figs. 1 to 3 are photographs of the hamburger patties of Comparative Examples 1 and 2 and Examples 1 and 2.

The hamburger patties of Comparative Examples 1 and 2 were similar in appearance (including texture) but the hamburger patty of Comparative Example 2 gave off a slightly stronger smell of meat due to the addition of salad oil.

The hamburger patty of Example 1 retained its meat shape and improved in appearance due to the addition of the short silk fibers. Furthermore, the hamburger patty of Example 1 provided a stringy mouthfeel like meat. On the other hand, the short silk fibers added to the hamburger patty of Example 1 stood out on the surface (for example, Fig. 1).

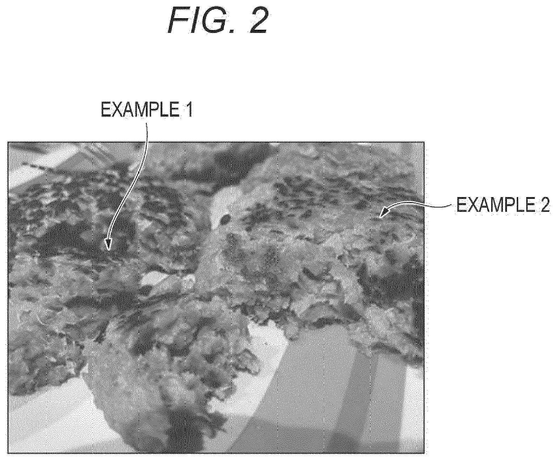

The hamburger patty of Example 2 did not get mushy, had a golden brown surface (for example, Fig. 2), and improved prominently in appearance. Furthermore, the short silk fibers played a role of connective tissues and settled the hamburger patty of Example 2 as compared with the hamburger patties of Comparative Example 2 and Example 1. Accordingly, the hamburger patty of Example 2 had an appearance and a mouthfeel much closer to those of real meat. In addition, the short silk fibers absorbed oil, which provided the hamburger patty with tenderness as a whole. The smell of the oil provided a meaty quality.

[Test Example 2: Preparation and Evaluation of Meat Substitute Composition]

<Example 3>

After a base sequence and an amino acid sequence of fibroin derived from Nephila clavipes (GenBank Accession Number: P46804.1, GI: 1174415) were acquired from the web database GenBank, amino acid residues were substituted, inserted, and deleted for the purpose of improving productivity, and a tag sequence and a hinge sequence were further added to the N-terminus, thereby designing recombinant fibroin having an amino acid sequence represented by SEQ ID NO: 1 (hereinafter also referred to as "PRT799").

Each nucleic acid that encodes the designed recombinant fibroin was synthesized. In the nucleic acid, an NdeI site was added to the 5' end and an EcoRI site was added downstream of the stop codon. This nucleic acid was cloned into a cloning vector (pUC118), cut out by being treated with restriction enzymes NdeI and EcoRI, and recombined into a protein expression vector pET-22b(+), thereby obtaining an expression vector.

Escherichia coli BLR (DE3) was transformed with the obtained expression vector. The transformed Escherichia coli was cultured for 15 hours in 2 mL of an LB culture medium containing ampicillin. The culture solution was added to 100 mL of a seed culture medium containing ampicillin (Table 6) so that $OD_{600}$ reached 0.005. The culture solution was maintained at a temperature of 30°C, and put in a culture flask (for about 15 hours) until $OD_{600}$ reached 5, thereby obtaining a seed culture solution.

[Table 6]

| Seed Culture Medium | |
|---|---|
| Reagent | Concentration (g/L) |
| Glucose | 5.0 |

(continued)

| Seed Culture Medium | |
|---|---|
| Reagent | Concentration (g/L) |
| $KH_2PO_4$ | 4.0 |
| $K_2HPO_4$ | 9.3 |
| Yeast Extract | 6.0 |
| Ampicillin | 0.1 |

The seed culture solution was added to a jar fermenter to which 500 mL of a growing medium (Table 7) had been added so that $OD_{600}$ reached 0.05. The culture solution was maintained at a temperature of 37°C and cultured while being controlled to have a constant pH of 6.9. In addition, a dissolved oxygen concentration in the culture solution was maintained at 20% of the saturated dissolved oxygen concentration.

[Table 7]

| Growing Medium | |
|---|---|
| Reagent | Concentration (g/L) |
| Glucose | 12.0 |
| $KH_2PO_4$ | 9.0 |
| $MgSO_4 \cdot 7H_2O$ | 2.4 |
| Yeast Extract | 15 |
| $FeSO_4 \cdot 7H_2O$ | 0.04 |
| $MnSO_4 \cdot 5H_2O$ | 0.04 |
| $CaCl_2 \cdot 2H_2O$ | 0.04 |
| ADEKA NOL (ADEKA, LG-295S) | 0.1 (mL/L) |

Immediately after glucose in the growing medium was completely consumed, a feed solution (455 g/1 L of glucose, 120 g/1 L of Yeast Extract) was added at a speed of 1 mL/min. The culture solution was maintained at a temperature of 37°C and cultured while being controlled to have a constant pH of 6.7. The culturing was performed for 20 hours while a dissolved oxygen concentration in the culture solution was maintained at 20% of the saturated dissolved oxygen concentration.

After that, a 1 M isopropyl-β-thiogalactopyranoside (IPTG) aqueous solution was added to the culture solution so that the final concentration reached 1 mM, thereby inducing expression of the target recombinant fibroin. The culture solution was centrifuged for 20 hours after the addition of IPTG, and bacterial cells were collected. SDS-PAGE was conducted using the bacterial cells prepared from the culture solution obtained before the addition of IPTG and after the addition of IPTG. The expression of the target recombinant fibroin that depended on the addition of IPTG was determined by the emergence of a band having the size of the target recombinant fibroin.

The bacterial cells collected 24 hours after the addition of IPTG were washed with a 20 mM Tris-HCl buffer (pH 7.4). The washed bacterial cells were suspended in a 20 mM Tris-HCl buffer (pH 7.4) containing about 1 mM phenylmethylsulfonyl fluoride (PMSF), and the bacterial cells were disrupted three times using a high-pressure homogenizer (Panda Plus 2000, GEA, available from GEA Niro Saovi Technologies). The disrupted cells were centrifuged (11,000 g, 10 minutes, at room temperature) with a centrifuge (Model 7000, available from Kubota Corporation) to obtain a precipitate. The obtained precipitate was washed with a 20 mM Tris-HCl buffer (pH 7.4) or 3% SDS buffer (pH 3.0) until the purity of the precipitate became high. The washed precipitate was suspended in a 8 M guanidine buffer (8 M guanidine hydrochloride, 10 mM sodium dihydrogen phosphate, 20 mM NaCl, 1 mM Tris-HCl, pH 7.0) so that the concentration of the precipitate reached 100 mg/mL, and then the precipitate was dissolved by being stirred with a stirrer at 60°C for 30 minutes. After the dissolving, the resultant was dialyzed with water using a dialysis tube (cellulose tube 36/32, available from Sanko Junyaku Co., Ltd.). A white aggregated protein obtained after the dialysis was collected by centrifugation, and water was removed by a freeze dryer to obtain lyophilized powder of the recombinant fibroin.

The lyophilized powder of the obtained recombinant fibroin was used to prepare a spinning solution (dope solution). To the lyophilized powder, 99% of formic acid was added so that the concentration of the lyophilized powder reached 24 mass%. After dissolving with a rotator for 14 hours, dust and bubbles were removed. The resultant was used as a spinning solution (dope solution).

The spinning apparatus illustrated in Fig. 4 was used to discharge the dope solution to a coagulation liquid (methanol) by a nitrogen air pump. Conditions of wet spinning were set in the following manner. Through the following process, recombinant fibroin fibers were obtained. <Wet Spinning Conditions> Dope solution temperature: 25°C, coagulation liquid (methanol) 1 temperature: 5°C, coagulation liquid (methanol) 2 temperature: 25°C, drawing bath temperature: 25°C, hot roller (HR) temperature: 60°C.

Using a mini warper (SW550, available from CCI TECH INC.), several hundreds of long fibers having a length of 3.6 m were obtained from a bobbin (derived from recombinant fibroin). The obtained long fibers were then cut with a powerful desktop fiber-cutting machine (NP-300, available from INTEC CO. LTD.) to obtain recombinant fibroin short fibers (about 100 g) having a length of 2 mm.

The obtained recombinant fibroin short fibers were dispersed and treated in sodium bicarbonate water for 10 minutes, followed by filtration. After that, the recombinant fibroin short fibers were washed several times with ion-exchanged water.

About 3 g of salad oil and 0.6 g of the washed recombinant fibroin short fibers were mixed into 60 g of the meat substitute composition of Comparative Example 1, thereby obtaining a meat substitute composition of Example 3. This meat substitute composition was picked up, shaped into a hamburger patty, and cooked in a frying pan, followed by sensory evaluation.

<Example 4>

A meat substitute composition of Example 4 was obtained in a manner similar to Example 3 except that an amount of washed recombinant fibroin short fibers to be mixed was changed from 0.6 g to 3 g. This meat substitute composition was picked up, shaped into a hamburger patty, and cooked in a frying pan, followed by sensory evaluation.

<Comparative Example 3>

A meat substitute composition of Comparative Example 3 was obtained in a manner similar to Comparative Example 2 of Test Example 1. This meat substitute composition was picked up, shaped into a hamburger patty, and cooked in a frying pan, followed by sensory evaluation.

<Comparative Example 4>

The meat substitute composition of Comparative Example 3 was picked up, shaped into hamburger patty, and cooked in a frying pan for a longer time than (twice as long as) the meat substitute compositions of Examples 3 and 4 and Comparative Example 3, followed by sensory evaluation.

(Sensory Evaluation)

Sensory evaluation was performed on hamburger patties of Comparative Examples 3 and 4 and Examples 3 and 4 to rate appearance, palatability, aroma, mouthfeel, and overall quality (overall meaty quality). The sensory evaluation was performed by two panels, and each property was rated on a scale of 1 to 4 (1: poor, 2: fair, 3: average, 4: good). Results are shown in Table 8.

[Table 8]

| Average score assigned by two panels | Appearance | Palatability | Aroma | Mouthfeel | Overall quality |
|---|---|---|---|---|---|
| Comparative Example 3 | 2.0 | 2.0 | 2.0 | 1.5 | 2.0 |
| Comparative Example 4 | 2.0 | 2.5 | 2.5 | 2.0 | 2.5 |
| Example 3 | 3.0 | 3.0 | 3.0 | 3.0 | 3.0 |
| Example 4 | 3.0 | 4.0 | 3.5 | 3.5 | 3.5 |

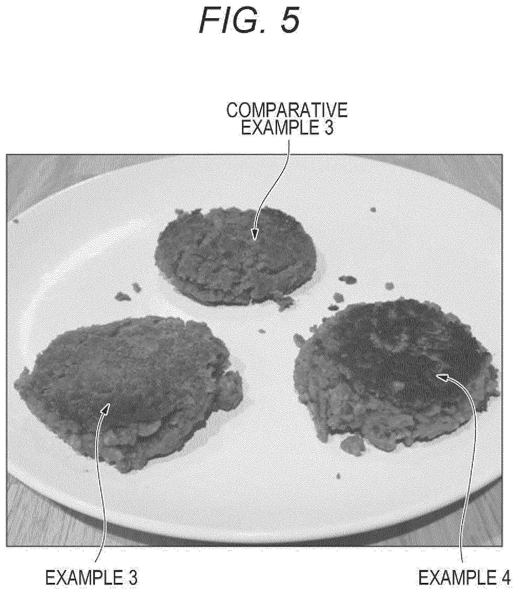

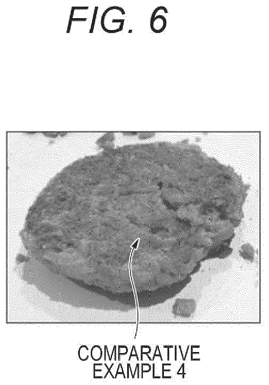

Figs. 5 and 6 are photographs of the hamburger patties of Comparative Examples 3 and 4 and Examples 3 and 4.

Due to the addition of the recombinant fibroin fibers, the hamburger patties of Examples 3 and 4 retained the appearance and overall shape and had no cracks on their brown surfaces. Surprisingly, the hamburger patties of Comparative Example 3 and Examples 3 and 4 had different browned surfaces (Fig. 5) even though the hamburger patties were cooked for the same period. Unlike the hamburger patty of Example 4, the hamburger patty of Comparative Example 4 was not browned (Fig. 6) even though it took twice as long to cook as the hamburger patties of Comparative Example 3 and Examples 3 and 4. The hamburger patties of Examples 3 and 4 improved in mouthfeel and did not get mushy compared with the hamburger patties of Comparative Examples 3 and 4.

In regard to the taste, the hamburger patties of Examples 3 and 4 did not evoke a dry and crumbly mouthfeel of the raw materials, unlike the hamburger patty of Comparative Example 3, and evoked a "stringy" mouthfeel like meat but not a "fibrous" feeling at the end. Furthermore, the hamburger patties of Examples 3 and 4 provided a meaty taste different from the hamburger patty of Comparative Example 3. The hamburger patties of Examples 3 and 4 had evaluation results similar to the results of the hamburger patty of Example 3 except that the recombinant fibroin short fibers did not stand out on the surface.

The hamburger patty of Example 4 to which 5 wt% of the recombinant fibroin short fibers were added was more savory, was not damaged (with no cracks on the surface), and had a less dry and crumbly mouthfeel.

[Test Example 3: Preparation and Evaluation of Meat Substitute Composition]

<Ingredients> (Raw Meat Material and Meat Substitute Composition Raw Material)

As a raw meat material or a meat substitute composition raw material, two types of ingredients, ground pork and OmniPork (available from OmniFoods), were prepared. OmniPork Ingredients List: water, protein blends (soy protein concentrate, soy protein isolate, shiitake fermented peas & rice protein), thickeners (methylcellulose, maltodextrin), yeast extract, palm oil, potato starch, cane sugar, salt, natural flavor (canola and sunflower oil), barley malt extract, colorant (beet red), dextrose, anti-caking agent (silicon dioxide).

(Polypeptide Fiber)

Lyophilized powder of the artificial fibroin PRT799 obtained in Example 3 was added to formic acid (available from ASAHI CHEMICAL CO., LTD.) so that the concentration of the lyophilized powder reached 31 mass%, and then the lyophilized powder was dissolved at 80°C. After that, dust and bubbles were removed to obtain a spinning solution 1 (dope solution 1).

To improve the level of hydrophobicity, all QQs in the amino acid sequence of SEQ ID NO: 1 were substituted with VFs and the remaining Qs with Is, thereby designing an amino acid sequence (amino acid sequence of SEQ ID NO: 2, PRT966).

A spinning solution was prepared using lyophilized powder of the recombinant fibroin PRT966 obtained in a manner similar to Example 3. To the lyophilized powder, 99% of formic acid was added so that the concentration of the lyophilized powder reached 26 mass%. After dissolving at 40°C, dust and bubbles were removed. The resultant was used as a spinning solution 2 (dope solution 2).

Similarly, the lyophilized powder of the obtained recombinant fibroin PRT966 was added to formic acid so that the concentration of the lyophilized powder reached 30 mass%, and then, the lyophilized powder was dissolved at 40°C. After that, dust and bubbles were removed to obtain a spinning solution 3 (dope solution 3).

The prepared dope solution 1, dope solution 2, or dope solution 3 was filled in a reserve tank. Using an inert gas (nitrogen), the spinning solution was discharged to a coagulation liquid (methanol) tank from a needle having an inside diameter with a pore size of 0.20 mm. Proteins were coagulated and washed and stretched in sequence with a methanol washing bath and a water washing bath, followed by drying with a dry heat plate, thereby obtaining recombinant fibroin fibers (original yarn) 1 to 3. Conditions of wet spinning were set in the following manner. Discharge pressure: 0.5 bar; draw ratio: 5 to 7 times; coagulation liquid temperature: 5 to 20°C; drying temperature: 60°C.

Diameters of the fibers were determined using a physical property measurement optical microscope. The stress and elongation of the protein fibers were measured at a temperature of 20°C and a relative humidity of 60% RH using a tensile tester (small desktop tester EZ-S, available from Shimadzu Corporation) to calculate the toughness. A sample was attached to a cardboard mold and a length between grippers was set to 20 mm, thereby performing tensile testing at a pulling speed of 10 mm/min. A load cell capacity was 1 N, and the grippers were of a clip type. The toughness was calculated based on the following Formula: $[E/(r^2 \times \pi \times L) \times 1000]$ (unit: MJ/m3) where "E" is fracture energy (unit: J), "r" is a fiber radius (unit: mm), "$\pi$" is pi, and "L" is a length between the grippers used in tensile testing (20 mm). The stress, elongation, and fiber diameter of each of the obtained recombinant fibroin fibers were evaluated, and each measured value was converted regarding physical values of the recombinant fibroin fiber 1 as 100%. Table 9 shows the results. Similarly, the stress, elongation, and fiber diameter of a commercially available soy fiber and a naturally derived silk fiber were evaluated. Table 9 shows the results.

[Table 9]

| | Stress (%) | Standard deviation of stress (%) | Strain (%) | Standard deviation of strain (%) | Fiber diameter ($\mu$m) |
|---|---|---|---|---|---|
| Recombinant fibroin fiber | 100 | 7.3 | 100 | 11.2 | 26.9 |
| Recombinant fibroin fiber | 153 | 8.2 | 62 | 15.4 | 10.7 |
| Recombinant fibroin fiber | 117 | 8.6 | 192 | 23.4 | 28.7 |
| Soy fiber | 253 | 25.6 | 54 | 26.3 | 13.9 |
| Naturally derived silk fiber | 310 | 30 | 58.4 | 32.6 | 11.9 |

The long fibers were cut with a powerful desktop fiber-cutting machine (NP-300, available from INTEC CO. LTD.) to a length of 3 mm, thereby obtaining about 20 g of recombinant fibroin short fibers 1 to 3 having a length of 3 mm.

(Preparation of Hamburger Patty)

As shown in Table 10, each of the obtained short fibers was added to the raw meat material or the meat substitute composition (Examples 5 to 8 and Comparative Examples 5 to 8) and kneaded for several minutes, whereby the fibers were dispersed uniformly. One hundred grams of the meat substitute composition was put in a circular mold having a diameter of 9 cm and a thickness of 1.8 cm to form a hamburger patty. The mold was covered with a lid, and the hamburger patty was stored at 4°C.

(Cooking and Evaluation of Hamburger Patty)

After 16 hours, a cooking paper sheet was put on an electric grill preset to 180°C, and the hamburger patty was cooked in the grill for seven minutes on each side.

[Table 10]

| | Raw meat material or meat substitute composition | Raw meat material content or meat substitute composition content (wt%) | Short fiber | Short fiber content (wt%) |
|---|---|---|---|---|
| Comparative Example 5 | Ground pork | 100 | - | - |
| Comparative Example 6 | OmniPork | 100 | - | - |
| Comparative Example 7 | OmniPork | 99 | Soy fiber | 1 |
| Comparative Example 8 | OmniPork | 99 | Naturally derived silk fiber | 1 |
| Example 5 | OmniPork | 99 | Recombinant fibroin fiber 1 | 1 |
| Example 6 | OmniPork | 99 | Recombinant fibroin fiber 2 | 1 |
| Example 7 | OmniPork | 99 | Recombinant fibroin fiber 3 | 1 |
| Example 8 | OmniPork | 95 | Recombinant fibroin fiber 2 | 5 |

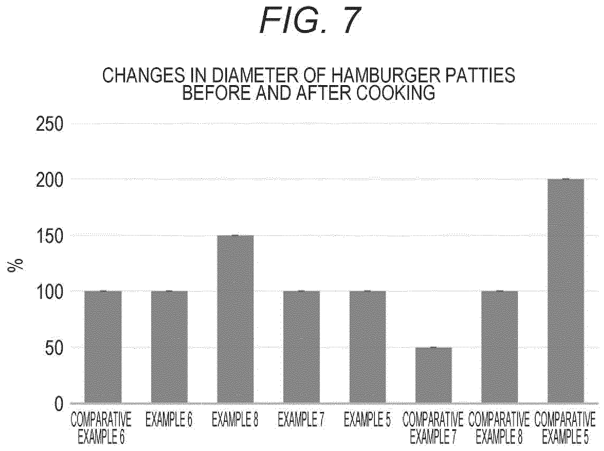

Changes in appearance (diameters of hamburger patties) that occurred in the cooking process were evaluated, and each measured value was converted regarding a rate of change in the hamburger patty of Comparative Example 6 as 100%. Fig. 7 shows the results. Changes in diameter of the hamburger patties before and after cooking were calculated from the following Formula: Percentage decrease in diameter (%) = [(diameter of raw hamburger patty) - (diameter after cooking)]/(diameter of hamburger patty) × 100%.

When the raw meat material was used (Comparative Example 5), proteins of animal meat were denatured and rapidly shrunk during cooking. When the meat substitute composition was used (Comparative Example 6), the phenomenon of shrinkage was not shown during the cooking process because the textured vegetable protein in the meat substitute composition was an already denatured protein. Adding 1 wt% of a protein fiber to the meat substitute composition did not cause a decrease in diameter, but adding 5 wt% of the recombinant fibroin fiber 2 (Example 8) increased the percentage decrease in diameter of the hamburger patty and showed a more realistic change in appearance during cooking.

[Test Example 4: Preparation and Evaluation of Meat Substitute Composition]

(Preparation, Cooking, and Evaluation of Hamburger Patty)

As shown in Table 11, the obtained short fibers were added to the raw meat material or the meat substitute composition (Examples 9 to 13 and Comparative Examples 9 to 12), whereby each hamburger patty was prepared as in Test Example 3, cooked, stored at 4°C and a relative humidity of 60%, and subjected to texture profile analysis.

[Table 11]

| | Raw meat material or meat substitute composition | Raw meat material content or meat substitute composition content (vol%) | Short fiber | Short fiber content (vol%) |
|---|---|---|---|---|
| Comparative Example 9 | Ground pork | 100 | - | - |
| Comparative Example 10 | OmniPork | 100 | - | - |
| Comparative Example 11 | OmniPork | 98.5 | Soy fiber | 1.5 |
| Comparative Example 12 | OmniPork | 98.5 | Naturally derived silk fiber | 1.5 |
| Example 9 | OmniPork | 98.5 | Recombinant fibroin fiber 1 | 1.5 |
| Example 10 | OmniPork | 99.5 | Recombinant fibroin fiber 2 | 0.5 |
| Example 11 | OmniPork | 98.5 | Recombinant fibroin fiber 2 | 1.5 |
| Example 12 | OmniPork | 97.5 | Recombinant fibroin fiber 2 | 2.5 |
| Example 13 | OmniPork | 98.5 | Recombinant fibroin fiber 3 | 1.5 |

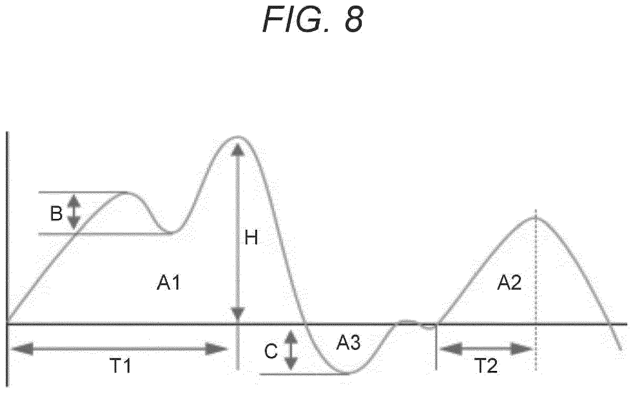

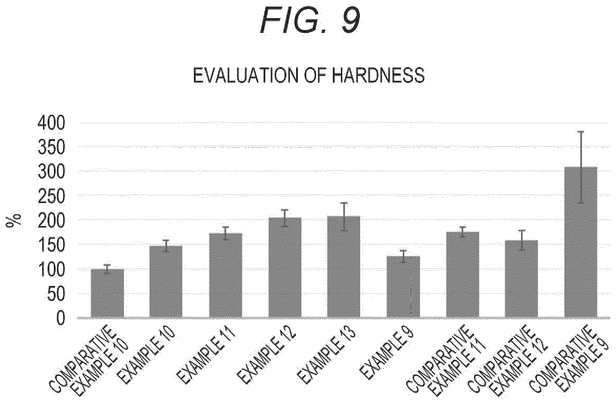

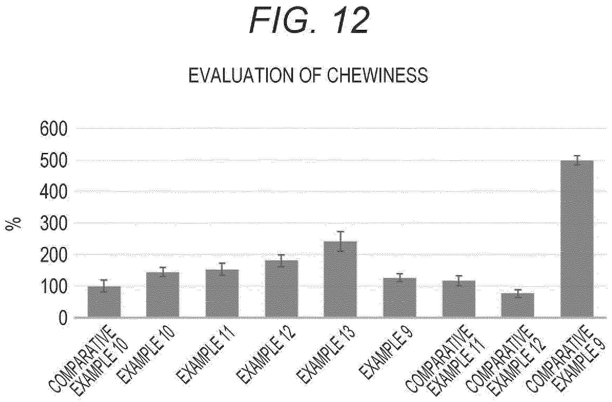

With regard to the texture of the cooked hamburger patties of Examples 9 to 13 and Comparative Examples 9 to 12, texture profile analysis was performed using Universal Testing Instrument EZ Test (available from Shimadzu Corporation). The cooked hamburger patty samples were cut to a size equal to original height × 2.5 cm × 2.5 cm and compressed to have a height 50% of the original height using a 500 N load cell. A crosshead speed was set to 300 mm/min, and a time interval between two compressions was set to two seconds. Each sample was measured nine times and subjected to texture profile analysis to comprehensively evaluate hardness, cohesiveness, springiness, and chewiness. Hardness, cohesiveness, springiness, and chewiness were calculated based on Fig. 8 and the following Formula: Each measured value was converted as 100% regarding a measured value of Comparative Example 10 without short fibers. Table 12 and Figs. 9 to 12 show the results.

Hardness: (H)
The maximum test force when a load is applied to food with a plunger

Cohesiveness: A2/A1

Application of a load to food deforms or damages the food

Ratio of areas (energy) between first and second loads applied continuously

Springiness: T2/T1

Ratio of "dent and displacement" between consecutive two loads applied to food with the plunger

Chewiness:

$$H \times A2/A1 \times T2/T1$$

Hardness × Springiness × Cohesiveness ... Solid food product

[Table 12]

| | Hardness (%) | Standard deviation of hardness (%) | Springiness (%) | Standard deviation of springiness (%) | Cohesiveness (%) | Standard deviation of cohesiveness (%) | Chewiness (%) | Standard deviation of chewiness (%) |
|---|---|---|---|---|---|---|---|---|
| Comparative Example 10 | 100 | 8 | 100 | 7 | 100 | 9 | 100 | 19 |
| Example 10 | 147 | 11 | 98 | 8 | 101 | 7 | 145 | 15 |
| Example 11 | 173 | 12 | 94 | 6 | 95 | 6 | 154 | 19 |
| Example 12 | 204 | 16 | 92 | 4 | 97 | 5 | 181 | 19 |
| Example 13 | 207 | 28 | 108 | 8 | 106 | 12 | 243 | 31 |
| Example 9 | 126 | 12 | 102 | 5 | 100 | 3 | 127 | 13 |
| Comparative Example 11 | 176 | 10 | 75 | 7 | 89 | 4 | 118 | 16 |
| Comparative Example 12 | 159 | 20 | 64 | 9 | 76 | 5 | 76 | 11 |
| Comparative Example 9 | 308 | 73 | 122 | 7 | 134 | 13 | 498 | 14 |

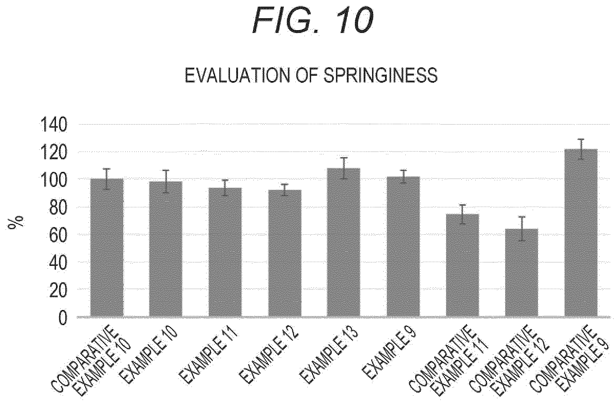

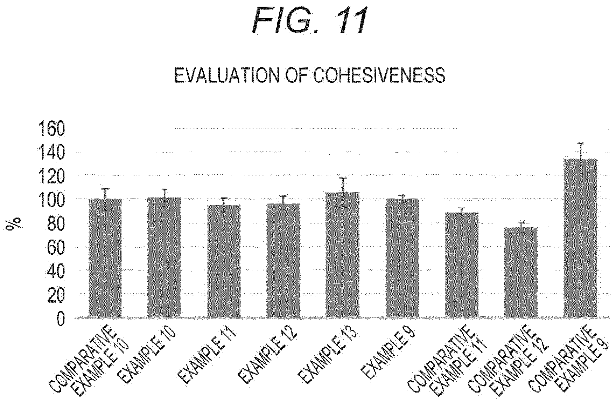

In adding 1.5 vol% of a fiber to the meat substitute composition, the addition of the soy fiber (Comparative Example 11) and the naturally derived silk fiber (Comparative Example 12) increased the hardness of the respective hamburger patties (Fig. 9) but significantly decreased the springiness and cohesion of the hamburger patties (Figs. 10 and 11). On the other hand, the addition of the recombinant fibroin fibers (Example 9, Example 11, and Example 13) increased the hardness of the respective hamburger patties (Fig. 9) without significantly decreasing the springiness and cohesion (Figs. 10 and 11).

When two types of proteins having different levels of hydrophobicity were used, the recombinant fibroin fiber having a high level of hydrophobicity (Examples 10 to 12) increased in hardness and chewiness as compared to the recombinant fibroin fiber having a low level of hydrophobicity (Example 9).

When two types of proteins having different mechanical properties were used, the recombinant fibroin fiber 3 having a fiber diameter of about 30 $\mu$m (Example 13) achieved a more realistic texture than that of the recombinant fibroin fiber 2 having a fiber diameter of about 10 $\mu$m (Example 11).

When an amount of the recombinant proteins added was increased to a range from 0.5 to 2.5 vol% (Example 10 to 12), the hamburger patty was enhanced in hardness without significantly decreasing the springiness and cohesion and provided a more realistic texture.

[Test Example 5: Preparation and Evaluation of Meat Substitute Composition]

(Preparation, Cooking, and Evaluation of Hamburger Patty)

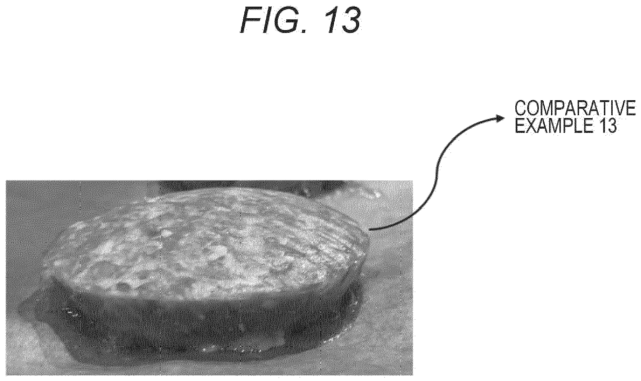

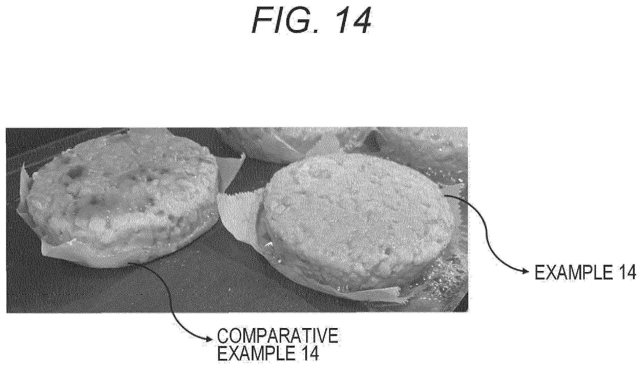

As shown in Table 13, the obtained short fibers were added to the raw meat material or the meat substitute composition (Example 14 and Comparative Examples 13 to 15), whereby each hamburger patty was prepared as in Test Example 3 and cooked. Figs. 13 and 14 show changes in appearance when cooking the first surface of each hamburger patty.

[Table 13]

| | Raw meat material or meat substitute composition | Raw meat material content or meat substitute composition content (wt%) | Short fiber | Short fiber content (wt%) |
|---|---|---|---|---|
| Comparative Example 13 | Ground pork | 100 | - | - |
| Comparative Example 14 | OmniPork | 100 | - | - |
| Example 14 | OmniPork | 95 | Recombinant fibroin fiber 2 | 5 |

When the raw meat material (Comparative Example 13) was used, moisture and fat ran down and were discharged during cooking, but, when the meat substitute composition (Comparative Example 14) was used, part of moisture was discharged to the upper surface and water droplets were formed on the surface. Adding the recombinant fibroin fiber to the meat substitute composition (Example 14) did not cause water droplets as in the hamburger patty using the raw meat material, but caused a more realistic change in appearance during cooking.

[Test Example 6: Preparation and Evaluation of Meat Substitute Composition]

(Preparation, Cooking, and Evaluation of Hamburger Patty)

As shown in Table 14, the obtained short fibers were added to the raw meat material or the meat substitute composition (Examples 15 to 13 and Comparative Examples 15 to 12) whereby each hamburger patty was prepared as in Test Example 3 and cooked.

[Table 14]

| | Raw meat material or meat substitute composition | Raw meat material content or meat substitute composition content (wt%) | Short fiber | Short fiber content (wt%) |
|---|---|---|---|---|
| Comparative Example 15 | Ground pork | 100 | - | - |
| Comparative Example 16 | OmniPork | 100 | - | - |
| Comparative Example 17 | OmniPork | 95 | Soy fiber | 5 |
| Comparative Example 18 | OmniPork | 95 | Naturally derived silk fiber | 5 |
| Example 15 | OmniPork | 95 | Recombinant fibroin fiber 1 | 5 |
| Example 16 | OmniPork | 95 | Recombinant fibroin fiber 2 | 5 |
| Example 17 | OmniPork | 95 | Recombinant fibroin fiber 3 | 5 |

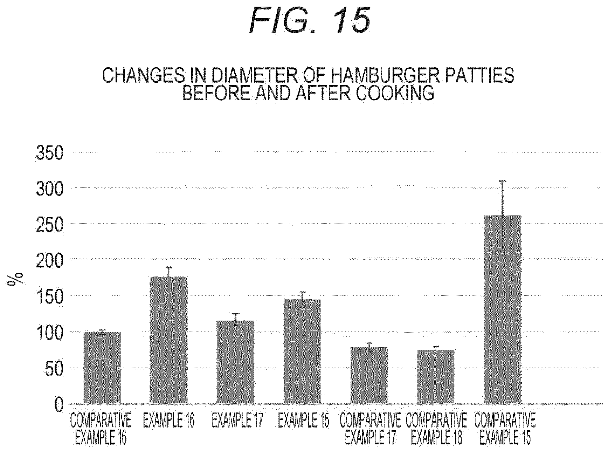

Changes in appearance (diameters of hamburger patties) that occurred in the cooking process were evaluated, and each measured value was converted as 100% regarding a rate of change in the hamburger patty of Comparative Example 16. Fig. 15 shows the results. Changes in diameter of the hamburger patties before and after cooking were calculated from the following Formula:

$$\text{Percentage decrease in diameter (\%)} = [(\text{diameter of raw hamburger patty}) - (\text{diameter after cooking})]/(\text{diameter of hamburger patty}) \times 100\%.$$

When the raw meat material was used (Comparative Example 15), proteins of animal meat were denatured and rapidly shrunk during cooking. When the meat substitute composition was used (Comparative Example 16), the phenomenon of shrinkage was not shown during the cooking process. Adding a soy fiber (Comparative Example 17) or naturally derived silk fiber (Comparative Example 18) to the meat substitute composition did not cause a decrease in diameter, but adding 5 wt% of the recombinant fibroin fibers 1 to 3 (Example 15 to 17) increased the percentage decrease in diameter of the respective hamburger patties and showed more realistic changes in appearance during cooking.

Reference Signs List

1    Extruder
2    Undrawn yarn-producing device
3    Wet-heat drawing device
4    Drying device
6    Dope solution
10    Spinning apparatus
20    Coagulation liquid tank
21    Drawing bath
36    Polypeptide fiber.

**Claims**

1. A meat substitute composition comprising a polypeptide that satisfies (1) or (2) below: (1) number of amino acid residues is 150 or more, an alanine residue content is 12 to 40%, and a glycine residue content is 11 to 55%; or (2) an amino acid residue content of at least one type selected from the group consisting of serine, threonine, and tyrosine, an alanine residue content, and a glycine residue content account for 56% or more altogether.

2. The meat substitute composition according to claim 1, wherein the polypeptide satisfies both (1) and (2).

3. The meat substitute composition according to claim 1 or 2, wherein the polypeptide is a recombinant polypeptide.

4. The meat substitute composition according to any one of claims 1 to 3, wherein the polypeptide has a plurality of repeating sequence units and the repeating sequence units each have 6 to 200 amino acid residues.

5. The meat substitute composition according to any one of claims 1 to 4, wherein the polypeptide contains an $(A)_n$ motif.

6. The meat substitute composition according to any one of claims 1 to 5, wherein the polypeptide is a structural protein.

7. The meat substitute composition according to any one of claims 1 to 6, wherein the polypeptide is fibroin.

8. The meat substitute composition according to any one of claims 1 to 7, wherein the polypeptide is in the form of a fiber.

9. The meat substitute composition according to any one of claims 1 to 8, further comprising a vegetable protein.

10. An artificial meat product comprising the meat substitute composition according to any one of claims 1 to 9 or a processed product of the meat substitute composition.

11. A method for making an artificial meat dish, the method comprising cooking the meat substitute composition according to any one of claims 1 to 9 or the artificial meat product according to claim 10.

## FIG. 1

COMPARATIVE
EXAMPLE 1

COMPARATIVE
EXAMPLE 2

EXAMPLE 1

# FIG. 2

EXAMPLE 1

EXAMPLE 2

# FIG. 3

(a)

(b)

(c)

COMPARATIVE
EXAMPLE 2

EXAMPLE 1

EXAMPLE 2

EP 4 098 127 A1

*FIG. 4*

## FIG. 5

COMPARATIVE
EXAMPLE 3

EXAMPLE 3

EXAMPLE 4

# FIG. 6

COMPARATIVE
EXAMPLE 4

# FIG. 7

CHANGES IN DIAMETER OF HAMBURGER PATTIES
BEFORE AND AFTER COOKING

FIG. 8

# FIG. 9

## EVALUATION OF HARDNESS

# FIG. 10

## EVALUATION OF SPRINGINESS

# FIG. 11

## EVALUATION OF COHESIVENESS

# FIG. 12

## EVALUATION OF CHEWINESS

# FIG. 13

COMPARATIVE
EXAMPLE 13

FIG. 14

EXAMPLE 14

COMPARATIVE
EXAMPLE 14

# FIG. 15

## CHANGES IN DIAMETER OF HAMBURGER PATTIES BEFORE AND AFTER COOKING

| | INTERNATIONAL SEARCH REPORT | International application No. |
|---|---|---|
| | | PCT/JP2021/003380 |

**A. CLASSIFICATION OF SUBJECT MATTER**

A23J 3/00(2006.01)i; A23J 3/14(2006.01)i; A23J 3/22(2006.01)i; C07K 14/00(2006.01)i; C07K 14/415(2006.01)i; C07K 14/435(2006.01)i; A23L 13/00(2016.01)i
FI: A23J3/00 502; A23J3/22; A23J3/14; A23J3/00 503; C07K14/00; C07K14/435; C07K14/415; A23L13/00 Z ZNA

**B. FIELDS SEARCHED**

Minimum documentation searched (classification system followed by classification symbols)
A23J3/00; A23J3/14; A23J3/22; C07K14/00; C07K14/415; C07K14/435; A23L13/00

Documentation searched other than minimum documentation to the extent that such documents are included in the fields searched

| | |
|---|---|
| Published examined utility model applications of Japan | 1922–1996 |
| Published unexamined utility model applications of Japan | 1971–2021 |
| Registered utility model specifications of Japan | 1996–2021 |
| Published registered utility model applications of Japan | 1994–2021 |

Electronic data base consulted during the international search (name of data base and, where practicable, search terms used)
JSTPlus/JMEDPlus/JST7580 (JDreamIII);
CAplus/MEDLINE/EMBASE/BIOSIS/WPIDS/FSTA (STN); UniProt/GeneSeq

**C. DOCUMENTS CONSIDERED TO BE RELEVANT**

| Category* | Citation of document, with indication, where appropriate, of the relevant passages | Relevant to claim No. |
|---|---|---|
| A | WO 2018/159695 A1 (KEIO GIJUKU) 07 September 2018 (2018-09-07) entire text | 1–11 |
| A | JP 2019-015709 A (ANDO, Hideo) 31 January 2019 (2019-01-31) entire text | 1–11 |
| A | CN 102972620 A (SOUTH CHIINA UNIVERSITY OF TECHNOLOGY) 20 March 2013 (2013-03-20) entire text | 1–11 |

☐ Further documents are listed in the continuation of Box C.   ☒ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 01 April 2021 (01.04.2021) | 13 April 2021 (13.04.2021) |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**INTERNATIONAL SEARCH REPORT**
Information on patent family members

| International application No. |
| --- |
| PCT/JP2021/003380 |

| Patent Documents referred in the Report | Publication Date | Patent Family | Publication Date |
| --- | --- | --- | --- |
| WO 2018/159695 A1 | 07 Sep.2018 | US 2020/0000091 A1<br>EP 3590339 A1<br>CN 110366370 A | |
| JP 2019-015709 A | 31 Jan. 2019 | US 2019/0008388 A1<br>CN 109211784 A | |
| CN 102972620 A | 20 Mar. 2013 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2014520554 A
- JP 2017517273 A
- JP 2009537177 A

**Non-patent literature cited in the description**

- *Nucleic Acid Res.,* 1982, vol. 10, 6487
- *Methods in Enzymology,* 1983, vol. 100, 448